# EUROPÄISCHE PATENTSCHRIFT

(11) **EP 0 902 649 B1**
(45) Veröffentlichungstag und Bekanntmachung des Hinweises auf die Patenterteilung: **05.09.2001**
(21) Anmeldenummer: 97923027.3
(22) Anmeldetag: 08.05.1997
(51) Int. Cl.: A61B 17/04

(54) **NAHTHILFEGERÄT**
SUTURING AID
DISPOSITIF D'AIDE A LA REALISATION DE SUTURES

(30) Priorität: 10.05.1996 DE 19618885; 31.08.1996 DE 19635354
(43) Veröffentlichungstag der Anmeldung: 24.03.1999
(73) Patentinhaber: Karl Storz GmbH & Co. KG, 78532 Tuttlingen (DE)
(72) Erfinder: BOCHE, Hartmut, D-88090 Immenstaad (DE); SCHERIEBLE, Hans, D-73734 Esslingen (DE)
(74) Vertreter: Weller, Wolfgang, Dr.rer.nat.
(86) Internationale Anmeldenummer: EP9702365
(87) Internationale Veröffentlichungsnummer: WO9742880

(56) Entgegenhaltungen:
- DE-C- 640 126
- US-A- 5 391 182
- US-A- 5 507 755
- US-A- 5 573 495

## Beschreibung

Die Erfindung betrifft ein Nahthilfegerät für das Verschließen von Minilaparotomien aus minimal-invasiven chirurgischen Eingriffen.

In der vergangenen Zeit hat die minimal-invasise Chirurgie eine weite Verbreitung gefunden. Insbesondere laparoskopische Operationen werden aufgrund der überragenden Vorteile für Patient und Chirurg nahezu ausschließlich mit der minimal-invasiven Chirurgietechnik durchgeführt.

Bei dieser Operationstechnik werden Führungshülsen, sogenannte Trokarhülsen, in denen ein angespitzter Dorn, ein sogenannter Trokardorn, aufgenommen ist, bspw. durch die Bauchdecke in den Bauchraum eingebracht. Nach Abziehen des Trokardorns können dann durch die Hülse Endoskope und Operationsinstrumente in das Operationsgebiet eingebracht werden. Bei komplizierten Operationen können mehrere Trokare an verschiedenen Stellen eingeführt werden. Unter visueller Kontrolle kann somit, im nur über kleine Stiche geöffneten Bauchraum, operiert werden.

Nach Beendigung der Operation werden die Instrumente und abschließend die Trokarhülse entfernt. Residual verbleiben kleine, jedoch die gesamte Bauchdecke, d.h. die Haut, die Unterhaut, die Muskelhaut, die Muskeln und das Bauchfell penetrierende Wunden, sogenannte Minilaparotomien.

Ursprünglich wurden bei dieser Operationstechnik die Minilaparotomien nicht verschlossen, was jedoch zur Bruchbildung, unter Umständen mit Einklemmung von Eingeweiden oder Eingeweidenteilen, führte, vergleichbar mit einem Leisten-, Nabel- oder Narbenbruch. Daher wurden Instrumente entwickelt, mit Hilfe derer Minilaparotomien verschlossen werden können.

Aus der US-A-5,474,568 ist ein Instrument zum Verschließen von Trokarwunden bekannt.

Dieses Instrument weist einen langen Schaft auf, an dessem proximalem Ende ein scherenartiger Handgriff angeordnet ist. Im Bereich des distalen Endes sind seitlich ausschwenkbare, etwa halbkreisförmig gebogene Nadeln vorgesehen, die einen Faden tragen. Der lange dünne Schaft wird durch die Trokarwunde hindurchgeschoben, dabei sind die Nadeln eingeschwenkt und überragen radial die Kontur des Schaftes nicht. Nach Einführen in den Körper werden die Nadeln seitlich ausgefahren und dringen von der Unterseite der Bauchdecke in diese ein, ziehen den Faden mit, bis die Spitze der gebogenen Nadel wieder den Schaftkörper erreicht und dort einrastet. Beim Zurückschwenken löst sich der eigentliche gebogene Nadelkörper von der eingerasteten Spitze nebst dem von dieser gehaltenen Faden. Das Gerät wird anschließend abgezogen und der Faden verknotet.

Dieses Instrument arbeitet somit als eigentliches Nahtgerät.

Nachteilig ist, daß der Operateur nicht feststellen kann, wann der Schaft bzw. dessen distaler Bereich gerade die gesamte Bauchdecke durchstoßen hat, jedoch noch nicht in andere, unter der Bauchdecke liegende Organe eingedrungen ist.

Es ist zu berücksichtigen, daß nach Abziehen der Trokarhülse keine Möglichkeit mehr besteht, visuell im Körper den Nahtvorgang zu beobachten und zu kontrollieren.

Ein ähnliches Nahtgerät mit seitlich ausschwenkbaren gebogenen Nadeln bzw. Nadelhaltern ist aus der US-A-5,364,408 bekannt.

Aus der DE-C-4 210 724 ist eine chirurgische Betätigungsvorrichtung bekannt, die ein langerstrecktes Schaftteil aufweist, aus dem Spreizteile in einer Ebene, senkrecht zur Schaftachse, seitlich ausfahrbar sind. Das Bewegen der in einer Radialebene spreizbaren Spreizteile erfolgt durch Drehen einer durch das Schaftteil hindurchgehenden Welle. In einer Ausführung ist vorgesehen, an den äußeren Spitzen der Spreizteile hochstehende Nadeln vorzusehen, die die Bauchdecke durchstoßen können. Auch dieses Gerät arbeitet als Nahtvorrichtung und beinhaltet die Gefahr, daß durch das radiale Ausfahren in einer Radialebene umliegende Organe beschädigt verletzt können.

Aus der DE-A-4 021 153 ist ein Organmanipulator bekannt, der einen Spreizkörper aus einem Mehrgelenk-Hebelsystem aus schwenkbeweglich miteinander verbundenen Gelenkarmen aufweist, die aus einer gestreckten Lage zum Einführen in die Körperhöhle in eine gespreizte Lage überführbar sind, wobei die Gelenkarme in ein dreieckförmiges Flächengebilde überführbar sind. Dieser Organmanipulator dient ausschließlich dazu, Organe in einer Körperhöhle freizulegen.

Aus der DE-A-640 126 ist ein Trokar mit einem Schaft beschrieben, an dessen distalem Ende zwei seitlich ausschwenkbare Schenkel angeordnet sind. Ein Betätigungselement dient zum Verschwenken der Schenkel, wobei die Schenkel zwischen einer seitlich ausgeklappten Arbeitsposition und einer proximal hochgeklappten Arbeitsposition steuerbar sind.

In der US-A-5 573 495 wird ein Gerät zum Anheben der Bauchwand beschrieben, das einen Schaft mit zwei seitlich ausschwenkbaren flächigen Auflagen und ein Betätigungselement zum Verschwenken der Auflagen aufweist. Die Auflagen sind dabei zwischen einer nach distal abgeklappten und einer seitlich ausgeklappten Arbeitsposition steuerbar.

Der Verschluß von Minilaparotomien ist somit bis zum gegenwärtigen Zeitpunkt technisch nicht zufriedenstellend gelöst.

Aufgrund des kleinen Hautschnittes, der in einem Durchmesserbereich von etwa 10 bis 15 mm liegt und der im Bereich von mehreren Zentimetern liegenden Dicken von Bauchdecken ist die Übersicht über alle Schichten der Bauchdecke, die verschlossen werden müssen, sehr schlecht. Der Verschluß von Bauchdeckenschichten ohne Sicht mit Nadel und Faden oder speziellen Klammern ist wegen bestehender Verletzungsgefahr von Baucheingeweiden risikoreich und nicht zu verantworten.

Aufgabe der vorliegenden Erfindung ist daher, ein Hilfsgerät zum Verschließen von Minilaparotomien zu schaffen, das dem Operateur das Anlegen der Naht erleichtert und Verletzungen beim Verschluß der Minilaparotomien verhindert.

Erfindungsgemäß wird die Aufgabe durch ein Nahthilfegerät gelöst, das einen Schaft aufweist, an dessen distalem Ende zumindest eine seitlich ausschwenkbare flächige Auflage angeordnet ist, sowie mit einem Betätigungselement zum Verschwenken der Auflage, wobei die Auflage zwischen einer nach proximal hochgeklappten Arbeitsposition, einer seitlich ausgeklappten Arbeitsposition und einer nach distal abgeklappten Arbeitsposition steuerbar ist.

Unter einer "flächigen Auflage" im Sinne der vorliegenden Erfindung wird ein jegliches flächiges Gebilde verstanden, sei es plattenartig, plattenförmig oder auch rost- oder gitterartige Konstruktionen, die letztendlich flächig bspw. an die Unterseite einer Bauchdecke angelegt werden können.

Der Begriff "Auflage" bedeutet nicht nur, daß auf dieser etwas aufgelegt werden kann, z.B. ein eigentliches Nahtgerät, sondern daß die Auflage auch an ein Körperteil angelegt werden kann.

Der Begriff "seitlich ausschwenkbar" im Sinne der vorliegenden Erfindung bedeutet, daß die Auflage aus der Längsachse des Schaftes seitlich herausbewegt bzw. herausgeklappt werden kann.

Unter dem Begriff "proximal hochgeklappte Arbeitsposition" wird eine Position verstanden, in der, von distal nach proximal längs der Schaftachse gesehen, das freie äußere Ende der Auflage näher am proximalen Ende liegt als deren Schwenkachse.

Unter dem Begriff "seitlich ausgeklappte Arbeitsposition" wird eine Stellung der Auflage verstanden, in der deren Fläche gegenüber der Schaftachse unter einem Winkel von etwa 90° steht.

Unter dem Begriff "distal abgeklappte Arbeitsposition" wird eine Schwenkstellung verstanden, bei der die Auflage gegenüber der zuerst genannten proximal hochgeklappten Arbeitsposition um mehr als 90° verschwenkt wurde, also deren äußeres Ende, von distal nach proximal gesehen, unter deren Schwenkachse zum Liegen kommt.

Durch diese Anordnung ist es nun möglich, die Auflage in eine eng an den Schaft angeschmiegte Position hochzuklappen, so daß der Zusammenbau aus Schaft und Auflage wie eine Art Pfeilspitze durch die Minilaparotomie eingeführt werden kann. Solange sich die Auflage noch in dem Wundkanal durch die Bauchdecke befindet, kann diese nicht seitlich ausgeschwenkt werden, was von dem Operateur einfach dadurch festgestellt werden kann, daß das Betätigungselement, das die Auflage verschwenkt, nicht bewegt werden kann.

Hat die Auflage z.B. die Bauchdecke durchstoßen, verläßt das oberste hochgeklappte Ende der Auflage als letztes Bauelement der Auflage den Wundkanal. Ab dieser Stellung kann die Auflage, von der Unterseite der Bauchdecke weg in Richtung Körperinnerem, relativ zum Schaft verschwenkt werden. Dadurch ist zum einen ausgeschlossen, daß zwischen der Unterseite der Bauchdecke und der Klappe Körperteile eingeklemmt werden, es ist sogar der gegenteilige Effekt zu erzielen, nämlich, daß durch diese von der Unterseite der Bauchdecke weg gerichteten Schwenkbewegung in Richtung Körperinnerem Organe von der Unterseite der Bauchdecke, die vernäht werden soll, weg bewegt werden. Dies ist atraumatisch durchzuführen. Der Operateur spürt diese Leichtgängigkeit sofort nachdem das oberste hochgeklappte Ende der Auflage den Wundkanal verlassen hat, weil er nämlich nunmehr das Betätigungselement zum Betätigen bzw. Ausschwenken der Auflage leicht betätigen kann. Ohne visuelle Beobachtung ist also für den Operateur einfach festzustellen, wann die Nahthilfe ausreichend tief durch den Wundkanal hindurchgeschoben ist.

Wurde nun die Auflage in die seitlich ausgeklappte definierte Arbeitsposition gebracht, kann das Nahthilfegerät in dieser Stellung etwas vom Körper weg bewegt werden, wodurch sich die Auflage dann flächig an die Unterseite der Bauchdecke bzw. des Gewebeabschnittes legt, durch den der Wundkanal hindurchreicht. Durch dieses Anheben kann dann die zu vernähende Gewebeschicht von darunterliegenden Körperorganen, bspw. bei einem Vernähen einer Bauchdecke, von den Eingeweiden abgehoben werden. Nun ist es möglich, von außen her Nadel und Faden längs des Schaftes einzuführen, und zwar soweit bis diese auf die Auflage treffen. Wird z.B. mit einem sogenannten Dechamps-Haken gearbeitet, der ein korkenzieherähnliches Gebilde ist, so kann dieser durch den Wundkanal längs des Schaftes des Nahthilfegerätes gewunden werden, bis dessen Spitze auf die Auflage trifft. Nunmehr kann der Haken, da er auch den Faden trägt, so von der Unterseite durch die Bauchdecke getrieben werden, daß an der gewünschten Stelle die Naht gelegt wird. Ohne visuelle Kontrolle ist also sichergestellt, daß der Dechamps-Haken oder andere Nadelhaken nicht zu tief in das Körperinnere eindringen und versehentlich innere Organe mit an die Unterseite der Bauchdecke annähen. Das Nahthilfegerät arbeitet also selbst nicht als Nahtgerät sondern hilft bzw. unterstützt an sich bekannte Nahtgeräte, wie z.B. das Anlegen einer Naht mit einem Dechamps-Haken.

Nach Legen des Fadens und Abziehen der Nadel bzw. des Dechamps-Hakens kann das Nahthilfegerät abgezogen werden, wobei die Auflage nach distal, also in Richtung Körperinnerem abgeklappt wird.

Dieses Abklappen hat den erheblichen Vorteil, daß nicht ein "Zurückklappen" in die hochgestellte Position erfolgt, wodurch die Gefahr besteht, daß zwischen hochklappender Auflage und Schaft Gewebeteile oder möglicherweise der Faden oder anderes eingeklemmt wird, sondern von der Unterseite des zu vernähenden Gewebes weg gerichtet verschwenkt wird. Das Anheben bzw. das Abziehen des Schaftes und die nach distal gerichtete Verschwenkbewegung kann synchron erfolgen, so daß unmittelbar zu Beginn des Abziehens des Nahthilfegerätes ein Abschnitt, also der Abschnitt der Auflage im Bereich der Anlenkstelle an den Schaft, in den Wundkanal eingezogen wird, somit nur ein relativ geringer Bereich der Auflage von der Unterseite der Bauchdecke weg in Richtung Körperinnerem bewegt wird, somit die Gefahr von Verletzungen ausgeschlossen ist. Körperteile in der Nähe der Auflage werden durch diese Schwenkbewegung von der Unterseite der zu vernähenden Gewebeschicht weg bewegt, somit wird die Gefahr ausgeschlossen, daß versehentlich Teile in den Wundkanal mit eingezogen werden.

Dadurch wird insgesamt gesehen dem Operateur ermöglicht, ohne genaue visuelle Kontrolle an exakter Stelle und ohne Beeinträchtigung umliegender Organe eine Naht zu legen. Dieser Vorgang bedarf nicht der hohen Aufmerksamkeit eines erfahrenen Operateurs, sondern kann auch von weniger erfahrenen oder geschulten Personen durchgeführt werden.

Das zuvor beschriebene Anheben des Nahthilfegerätes bei seitlich ausgeklappter Auflage ermöglicht nicht nur ein Abheben der zu vernähenden Schicht, bspw. der Bauchdecke von inneren Organen, sondern führt auch zu einem leichten Spreizen des Wundkanals, so daß Teile der Fläche der Auflage von außen ersichtlich sind, wodurch besonders leicht bspw. ein Dechamps-Haken eingesetzt werden kann.

Somit wird die Aufgabe vollkommen gelöst.

In einer besondere Ausgestaltung der Erfindung weist die Auflage zwei diametral gegenüberliegende Flügelplatten auf.

Diese Maßnahme hat nun den Vorteil, daß durch die zwei Flügelplatten beidseits des Schaftes flächige Auflagen geschaffen werden, so daß bspw. links und rechts des Schaftes jeweils eine Naht gesetzt werden kann, was schon ausreichend ist, um Minilaparotomien zu verschließen. Die beiden Flügelklappen bilden beim Einschieben ein pfeilspitzenartiges "V", das beim Abziehen entsprechend dann nach unten geöffnet ist. Dieses nach unten offene V beim Abziehen stellt sicher, daß keine Gewebeteile eingeklemmt und mit abgezogen werden können.

In einer weiteren Ausgestaltung der Erfindung ist das Betätigungselement als längs des Schaftes verschiebbarer Stab ausgebildet, der distal gelenkig mit der Auflage verbunden ist, wobei die Gelenkachse die Schwenkachse der Auflage darstellt.

Diese Maßnahme hat zum einen den konstruktiven Vorteil, daß mit wenigen einfachen, und somit auch einfach zu reinigenden Bauelementen ausgekommen wird, die letztendlich in einer äußerst schlanken Bauweise des Nahthilfegerätes resultiert.

In einer weiteren Ausgestaltung der Erfindung ist die Auflage im Abstand zu ihrer Schwenkachse gelenkig mit einem Ende eines Betätigungshebels verbunden, dessen anderes Ende gelenkig mit dem Schaft verbunden ist.

Diese Maßnahme hat den Vorteil, daß durch die Hebelanordnung die axiale Verschiebung des Betätigungselementes längs des Schaftes zielsicher in die gewünschte Schwenkbewegung der Auflage umgesetzt wird.

In einer weiteren Ausgestaltung der Erfindung entspricht der Abstand zwischen der Schwenkachse der Auflage und der Anlenkung des Hebels in etwa einem Drittel der Gesamtlänge der Auflage.

Diese Hebelgeometrie erlaubt ein funktionssicheres Umsetzen der Verschiebebewegung des Betätigungselementes in die Verschwenkbewegung der Auflage ohne großen Kraftaufwand.

In einer weiteren Ausgestaltung der Erfindung ist auf der dem proximalen Ende des Nahthilfegerätes zugewandten Seite der Auflage zumindest eine Führung für ein Nahtwerkzeug vorgesehen.

Diese Maßnahme hat den Vorteil, daß das Nahtwerkzeug, wenn es die Auflagefläche erreicht hat, etwas hin- und hergeschoben werden kann, bis es in die Führung, bspw. eine Führungsnut, eintritt. Dies spürt der Operateur und weiß dann, daß sich das Nahtwerkzeug an einer bestimmten Stelle auf der Auflage befindet. Er kann dadurch das Nahtwerkzeug ausgerichtet an der zu nähenden Stelle ansetzen und ohne visuelle Kontrolle der Spitze des Nahtwerkzeuges exakt den Faden legen.

In einer weiteren Ausgestaltung der Erfindung ist das Betätigungselement verdrehsicher im Schaft angebracht.

Diese Maßnahme hat den Vorteil, daß das Betätigungselement in einer ganz bestimmten definierten Drehstellung zum Schaft verbleibt, somit der Operateur weiß, in welcher Stellung bzw. in welcher Erstreckung sich die Auflage befindet, selbst dann, wenn diese in den Körper eingeschoben ist und vom Operateur nicht mehr ersichtlich ist.

In einer weiteren Ausgestaltung der Erfindung ist das Betätigungselement durch die Kraft einer Feder derart beaufschlagt, daß die Auflage aus der nach proximal hochgeklappten Arbeitsposition selbständig in Richtung der seitlich ausgeklappten Arbeitsposition bewegt wird.

Diese höchst bevorzugte Ausgestaltung hat den erheblichen Vorteil, daß die Auflage, nachdem sie durch den Wundkanal hindurchgeschoben wurde, selbständig seitlich ausschwenkt, wodurch auch das Betätigungselement bewegt wird. Dies kann der Operateur von außen erkennen, so daß er genau weiß, daß er nun die Auflage durch den Wundkanal hindurchgeschoben hat. Damit wird besonders einfach sichergestellt, daß das Nahthilfegerät nicht versehentlich zu weit eingetrieben wird.

In einer weiteren Ausgestaltung der Erfindung ist die Kraft der Feder derart eingestellt, daß die Arbeitsauflage nur bis in eine Zwischenposition seitlich ausgeklappt wird, wobei die Zwischenposition etwas vor der vollständig seitlich ausgeklappten Arbeitsposition liegt.

Diese Maßnahme hat den Vorteil, daß der Operateur, nachdem er erkannt hat, daß die hochgeklappte Auflage den Wundkanal durchschritten hat, nunmehr durch geringfügiges Zurückziehen des Nahthilfegerätes die Auflage in die gewünschte, meist um exakt 90° seitlich ausgeklappte Arbeitsposition bringen kann, in der diese dann festgestellt werden kann.

In einer weiteren Ausgestaltung der Erfindung sind am Schaft und/oder am Betätigungselement Orientierungsmerkmale vorgesehen, die die jeweilige Schwenkstellung der Arbeitsauflage anzeigen.

Diese Maßnahme hat nun den Vorteil, daß der Operateur durch die Relativstellung zwischen Schaft und längs diesem beweglichen Betätigungselement zu jedem Zeitpunkt erkennen kann, auch ohne visuellen Kontakt mit der Auflage, in welcher Schwenkstellung diese sich befindet. Dies trägt erheblich zur Sicherheit der Handhabung des Nahthilfegerätes bei.

In einer weiteren Ausgestaltung der Erfindung ist ein Feststellmechanismus vorgesehen, über den die Arbeitsauflage in bestimmten Schwenkstellungen feststellbar ist.

Diese Maßnahme hat den Vorteil, daß über den Feststellmechanismus die Auflage, insbesondere in ihrer seitlich ausgeschwenkten Arbeitsposition, feststellbar ist, so daß dann die weiteren Manipulationen, wie das Setzen und Legen des Fadens mit einem Dechamps-Haken, durchgeführt werden können, ohne daß sich die Relativstellung zwischen Auflage und Schaft ändert.

In einer weiteren Ausgestaltung der Erfindung ist der Feststellmechanismus als ein am Schaft schwenkbar gelagerter Federhebel ausgebildet, der ein Rastelement aufweist, das in entsprechende Aussparungen am Betätigungselement eingreift.

Diese Maßnahme hat nicht nur den Vorteil, daß der Feststellmechanismus einfach zu betätigen, also zu schließen oder zu lösen ist, sondern daß zugleich auch erkannt werden kann, welche Stellung gerade festgestellt wird.
In einer weiteren Ausgestaltung der Erfindung sind die Aussparungen mit Rampen versehen, die ein erkennbares Einrasten verursachen.

Diese Maßnahme hat nun den erheblichen handhabungsmäßigen Vorteil, daß der Operateur entweder durch ein hörbares Klicken oder ersichtliches Bewegen des Feststellmechanismus erkennt, ob die Auflage festgestellt ist oder nicht.

In einer weiteren Ausgestaltung der Erfindung ist vorgesehen, daß zur Einführung des Nahthilfegerätes in den Wundbereich eine spezielle Führungsschiene vorgesehen ist.

Diese Maßnahme hat den Vorteil, daß das Nahthilfegerät gezielt längs der Führung in den Wundkanal ein- bzw. durchgeführt werden kann.

In einer weiteren Ausgestaltung der Erfindung weist die Führungsschiene einen abgewinkelten Griff auf.
Diese Maßnahme hat den Vorteil, daß das Halten der Führungsschiene bei gleichzeitig angelegtem Nahthilfegerät einfach möglich ist.

In einer weiteren Ausgestaltung der Erfindung weist die Führungsschiene eine Führungsnut für einen Führungszapfen am distalen Ende des Betätigungselementes auf, der eine spürbare Führung und Steuerung der Eindringtiefe des Nahthilfegerätes bezogen auf die Eindringtiefe der Führungsschiene erlaubt.

Diese Maßnahme hat den Vorteil, daß eine ganz definierte Führung und Steuerung beim Einschieben des Nahthilfegerätes möglich ist.

In einer weiteren Ausgestaltung der Erfindung weist die Führungsschiene eine Skala auf.

Diese Maßnahme hat den Vorteil, daß über die Skala die Einschubtiefe der Führungsschiene kontrolliert werden kann. Die Einschubtiefe des Trokars kann man erkennen und erfassen und dann dementsprechend tief die Führungsschiene einschieben. Diese ist abhängig von der jeweiligen Körperbeschaffenheit des Patienten. Dadurch ist sichergestellt, daß das Einbringen der Führungsschiene keine Organe verletzt.

Es versteht sich, daß die vorstehend genannten und die nachstehend noch zu erläuternden Merkmale nicht nur in ihren angegebenen Kombinationen sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert.

Es zeigen:
- Fig. 1: eine perspektivische Ansicht eines ersten Ausführungsbeispieles eines Nahthilfegerätes,
- Fig. 2: eine teilweise schematisierte Ansicht des distalen Endbereiches des Nahthilfegerätes in einer proximal hochgeklappten Arbeitsposition der Auflage beim Durchschieben durch eine Minilaparotomie,
- Fig. 3: eine der Fig. 2 vergleichbare Darstellung in einer schon seitlich ausgeklappten Zwischenposition der Auflage,
- Fig. 4: eine den Darstellungen von Fig. 2 und 3 vergleichbare Darstellung in der seitlich vollständig ausgeklappten Arbeitsposition der Auflage,
- Fig. 5: eine den Darstellungen von Fig. 2 bis 4 vergleichbare Ansicht mit einer nach distal abgeklappten Arbeitsposition der Auflage während des Abziehens aus einer Minilaparotomie,
- Fig. 6: eine perspektivische Ansicht von proximal nach distal des distalen Endbereiches des Nahthilfegerätes in einer Stellung entsprechend der Stellung von Fig. 2,
- Fig. 7: eine der Darstellung von Fig. 3 entsprechende perspektivische Darstellung,
- Fig. 8: eine der Darstellung von Fig. 4 entsprechende perspektivische Darstellung,
- Fig. 9: eine der Darstellung von Fig. 5 entsprechende perspektivische Darstellung,
- Fig. 10: eine Draufsicht von proximal nach distal auf eine Minilaparotomie mit bereits eingesetztem Nahthilfegerät in der Spreizstellung von Fig. 4,
- Fig. 11: eine Seitenansicht einer Führungsschiene zum Einführen des Nahthilfegerätes in die Minilaparotomie,
- Fig. 12: eine um 90° verdrehte Seitenansicht der Führungsschiene von Fig. 6 mit angesetztem Nahthilfegerät von Fig. 1,
- Fig. 13: ein stark vergrößerter Querschnitt der Führungsnut der Führungsschiene von Fig. 11, wobei diese an einen Trokar angelegt ist,
- Fig. 14: eine der Schnittdarstellung von Fig. 13 vergleichbare Schnittdarstellung mit angelegtem Nahthilfegerät,
- Fig. 15: eine Seitenansicht eines weiteren Ausführungsbeispiels eines Nahthilfegerätes in Kunststoffversion,
- Fig. 16: eine vergrößerte Darstellung des Nahthilfegerätes von Fig. 15 im distalen Bereich,
- Fig. 17: einen Schnitt längs der Linie XVII-XVII in Fig. 16 und
- Fig. 18: eine der Schnittdarstellung von Fig. 10 vergleichbare Darstellung des Nahthilfegerätes in seitlich ausgeklapptem Zustand.

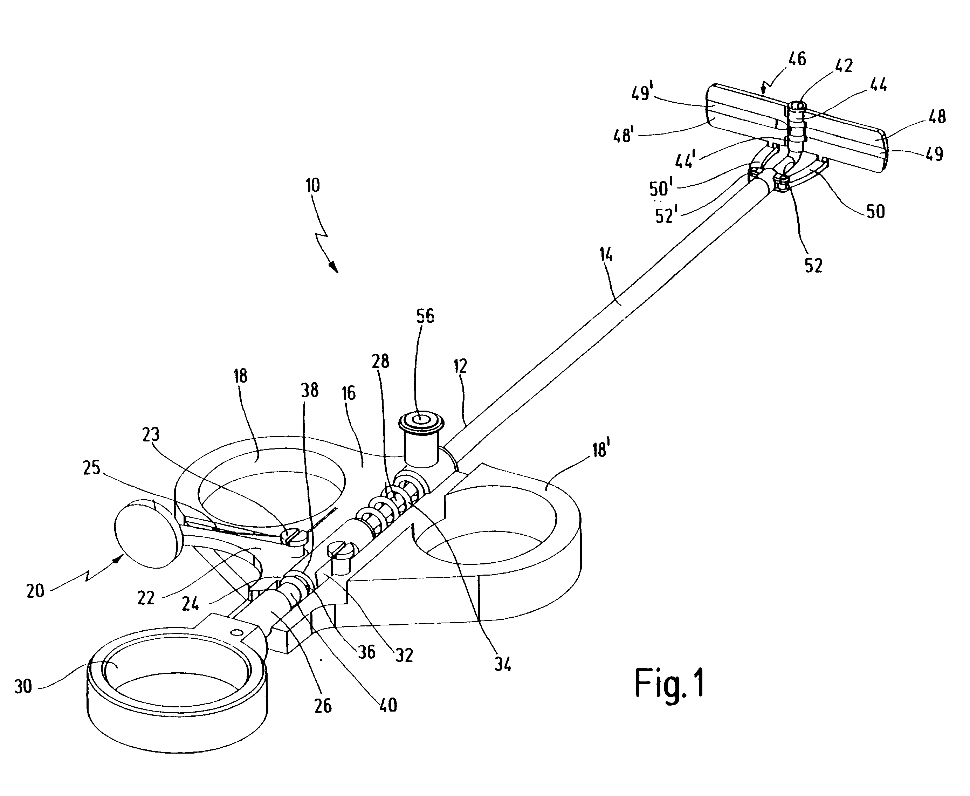

Ein in Fig. 1 perspektivisch dargestelltes erstes Ausführungsbeispiel eines Nahthilfegerätes ist in seiner Gesamtheit mit der Bezugsziffer 10 versehen.

Das Nahthilfegerät 10 weist einen langerstreckten Schaft 12 in Form eines Rohres 14 auf.

Am proximalen Ende des Rohres 14 ist ein Griff 16 vorgesehen, der zwei sich diametral gegenüberstehende, etwa ringförmige, sich in einer Ebene erstreckende Fingerösen 18, 18' aufweist.

Am Griff 16 ist ein Feststellmechanismus 20 vorgesehen, der einen Federhebel 22 aufweist.

Der Federhebel 22 ist um eine am Griff 16 ortsfeste Schwenkachse 23 schwenkbar angebracht und weist an einem Ende einen Zapfen 24 auf, der, wie später beschrieben wird, in entsprechende Aussparungen bzw. Nuten sperrend eingreift.

Der Federhebel 22 ist durch eine Feder 25 beaufschlagt, die diesen in Feststell- bzw. Verriegelungsstellung drückt. An dem dem Zapfen 24 gegenüberliegenden Ende ist der Federhebel 22 mit einer hier nicht näher bezeichneten Taste versehen, die von einer Fingerkuppe betätigt werden kann.

Im Rohr 14 des Schaftes 12 ist ein Betätigungselement 26 in Form eines Stabes 28 längsverschieblich aufgenommen.

Der Stab 28 ist an seinem den Griff 16 proximal überragenden Ende mit einer Fingeröse 30 versehen, die sich in derselben Ebene wie die Fingerösen 18, 18' erstreckt.

In dem in Fig. 1 ersichtlichen Abschnitt des Stabes 28 im Bereich des Griffes 16 ist dieser mittig mit einer seitlichen Abflachung 32 versehen, die einerseits dazu dient, daß der Stab 28 verdrehsicher im Rohr 14 aufgenommen ist, wozu entsprechende Klemmelemente vorgesehen sind.

Die der in Fig. 1 ersichtlichen Abflachung 32 diametral gegenüberliegenden Abflachung dient gleichzeitig als Gleitfläche für den Zapfen 24 des Federhebels 22, wie das nachfolgend noch näher in Zusammenhang mit der Funktionsweise beschrieben wird.

Im Abstand zur Abflachung 32 ist proximal dazu eine erste Ringnut 36 eingeschnitten, deren Flanken als Rampen 38 ausgebildet sind.

An die relativ schmale Nut 36 schließt sich proximal eine relativ breite Nut 40 an, deren Flanken ebenfalls als Rampen ausgebildet sind.

Der Zapfen 24 ist so ausgebildet, daß er sowohl in die Nut 36 als auch in die breite Nut 40 einrasten kann.

Am distalen Ende weist der Stab 28 ein abgewinkeltes Ende 42 auf, auf dem scharnierförmige Gelenke 44 einer Auflage 46 aufgenommen sind.

Die Auflage 46 besteht aus zwei diametral gegenüberliegenden, etwa rechteckförmigen Flügelplatten 48, 48', die somit schwenkbar um das abgewinkelte Ende 42 des Stabes 28 montiert sind. Die Mittellängsachse des abgewinkelten Endes 42 stellt somit die Gelenkachse 45 bzw. die entsprechende Schwenkachse der Flügelplatten 48, 48' dar. Auf der dem proximalen Ende des Nahthilfegerätes 10 zugewandten Seite sind die Flügelplatten 48, 48' mittig mit einer sich längs der Längsachse erstreckenden Führungsnut 49 bzw. 49' versehen.

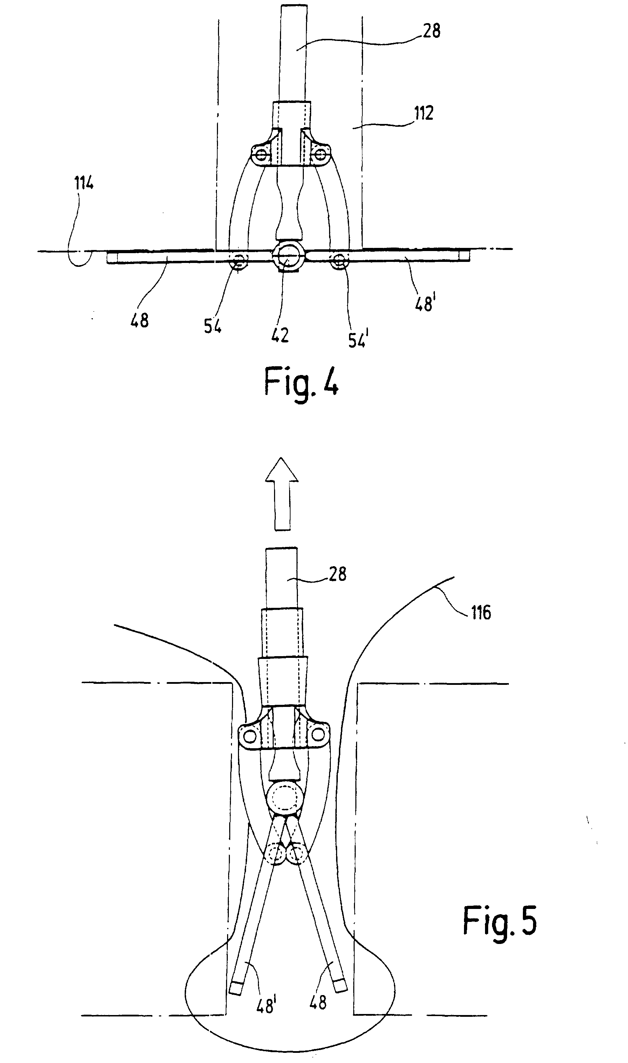

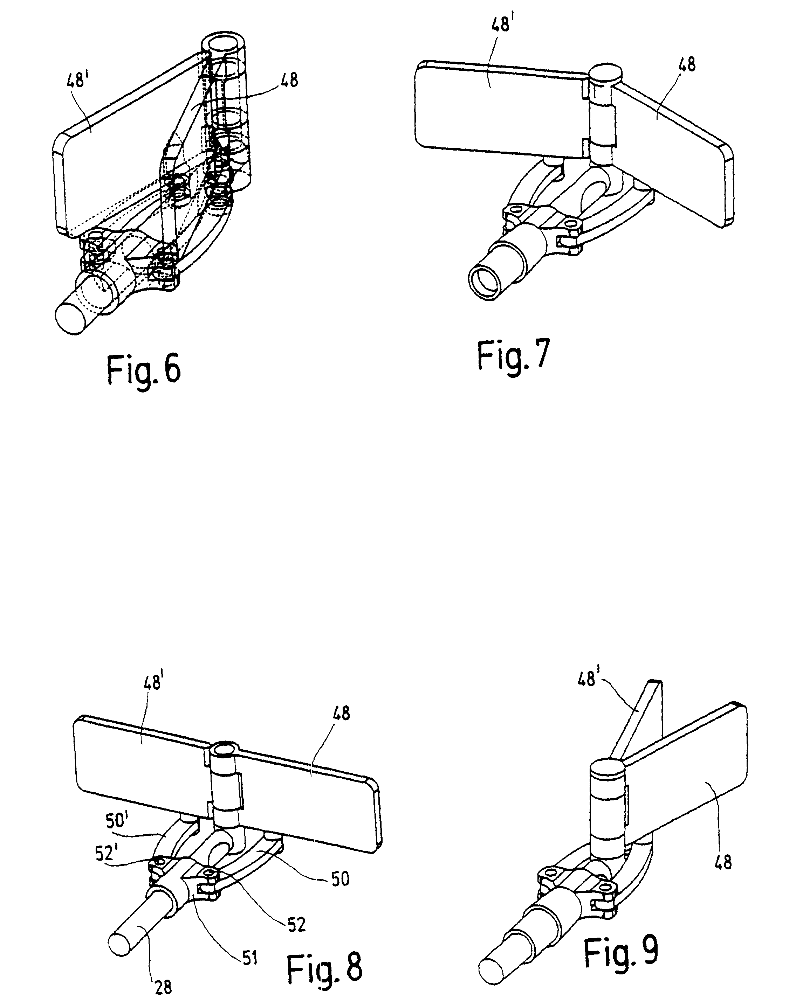

Die Flügelplatten 48, 48' sind über Betätigungshebel 50, 50' gelenkig mit einem Flansch 51 am distalen Ende des Schaftes 12 verbunden. Der Betätigungshebel 50 ist, wie das insbesondere auch in Zusammenhang mit Fig. 4 und 8 ersichtlich ist, über einen Gelenkbolzen 52 einerseits gelenkig mit dem Flansch 51 und andererseits über ein Gelenkbolzen 54 gelenkig mit einer Seitenkante der Flügelplatte 48 verbunden. Der Abstand zwischen Gelenkachse 45 und Gelenkbolzen 54 beträgt etwa ein Drittel der axialen Länge der Flügelplatte 48. Entsprechendes gilt dann für die gelenkige Verbindung der Flügelplatte 48' mit dem Betätigungshebel 50'.

Im Bereich des Griffs 16 ist am Schaft 12 ein radial vorspringender Stutzen 56 vorgesehen, der zur Reinigung und zur Spülung des hohlen Schaftes 12 dient.

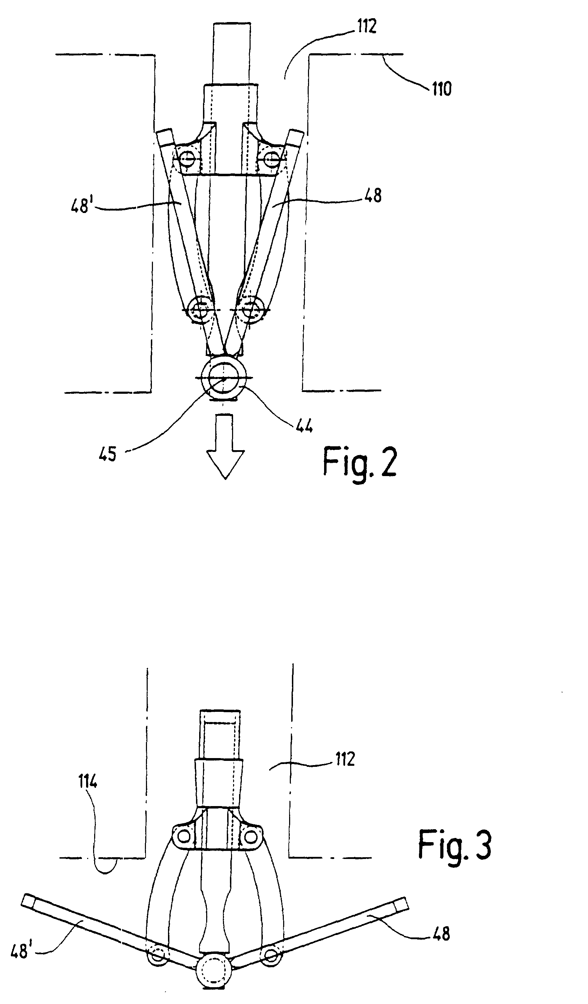

Die verschiedenen Arbeitspositionen und Schwenkstellungen der Flügelplatten 48, 48' sollen nachfolgend anhand der Bildfolge von Fig. 2 bis 5 beschrieben werden, wobei die Bildfolge von Fig. 6 bis 9 entsprechende perspektivische Darstellungen der Schwenkstellung der Flügelplatten 48, 48' zeigt.

Die Grundstellung der Flügelplatten 48, 48', also so wie dem Operateur das Nahthilfegerät 10 gereicht wird, ist die in Fig. 1 bzw. in Fig. 8 dargestellte Stellung. In dieser Darstellung sind die Flügelplatten 48, 48' etwa um 90° aus der Schaftachse verschwenkt und erstrecken sich in ein und derselben Ebene.

In dieser Stellung ist der Zapfen 24 des Feststellmechanismus 20 in die schmale Nut 36 des Stabes 28 eingerastet, somit sind die Flügelplatten 48, 48' in dieser Schwenkstellung unverrückbar.

Der Operateur ergreift nunmehr das Nahthilfegerät 10, schiebt zwei Finger in die Fingerösen 18, 18', bspw. den Zeigefinger und Mittelfinger. Mit dem Daumen kann nun der Feststellmechanismus 20 gelöst werden, so daß das Nahthilfegerät 10 mit seinem distalen Ende voran in eine Minilaparotomie 112, bspw. in einer Bauchdecke 110, eingeschoben werden kann, wie das in Fig. 2 dargestellt ist. Die Fingeröse 30 braucht dazu nicht ergriffen werden.

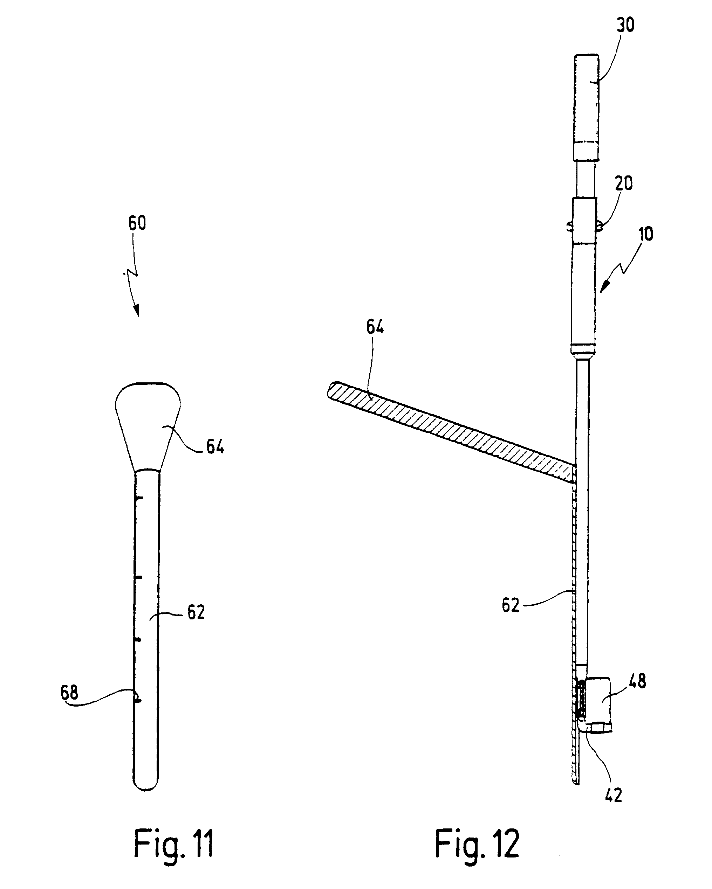

Ein Vorgang zum Erleichtern dieses Einführens wird später in Zusammenhang mit den Fig. 11 bis 14 beschrieben.

Der Durchmesser der Minilaparotomie 112 ist wesentlich geringer als die radiale Erstreckung der ausgeklappten Flügelplatten 48, 48', so daß diese nach proximal hochgeklappt werden und dabei eine V-förmige Position einnehmen, wie sie in Fig. 2 und 6 dargestellt ist. Dabei wird der Stab 28 gegen die Kraft der Feder 34 im Schaft 12 nach distal verschoben, die Fingeröse 30 bewegt sich also auf den Griff 16 zu. Dies kann entweder vom Operateur unterstützt werden, dieser Vorgang wird aber auch von den Wänden der Minilaparotomie 112 bewerkstelligt, ohne daß dadurch eine Verletzung oder eine Aufweitung der Minilaparotomie 112 erfolgt. Der Operateur kann also bspw. den Daumen aus der Fingeröse 30 bei diesem Vorgang herausnehmen.

Wurde das distale, in der Zwischenzeit V-förmige Ende der hochgeklappten Flügelplatten 48, 48' durch die Minilaparotomie 112 hindurchbewegt, wie das aus dem Übergang von Fig. 2 zu Fig. 3 ersichtlich ist, drückt die Kraft der Feder 34 den Stab 28 im Schaft nach proximal. Über die Betätigungshebel 50, 50' werden die Flügelplatten 48, 48' seitlich ausgeschwenkt, bis sie etwa die in Fig. 3 ersichtliche Zwischenposition erreicht haben. In diesem Bereich ist der Zapfen 24 des Federhebels 22 des Feststellmechanismus 20 längs der breiten Nut 40 gegleitet und ist nun an dessen distaler Flanke, die als Rampe ausgebildet ist, angeschlagen.

Der Übergang aus der Stellung von Fig. 2 zu Fig. 3 erfolgt durch die Kraft der Feder 22 ruckartig, wobei dies mit einer ruckartigen Bewegung der Fingeröse 30 verbunden ist, die der Operateur erkennen kann, ihm also angezeigt ist, daß nunmehr die Flügelplatten 48, 48' durch die Minilaparotomie 112 in den freien Bauchraum durchgeschoben worden sind.

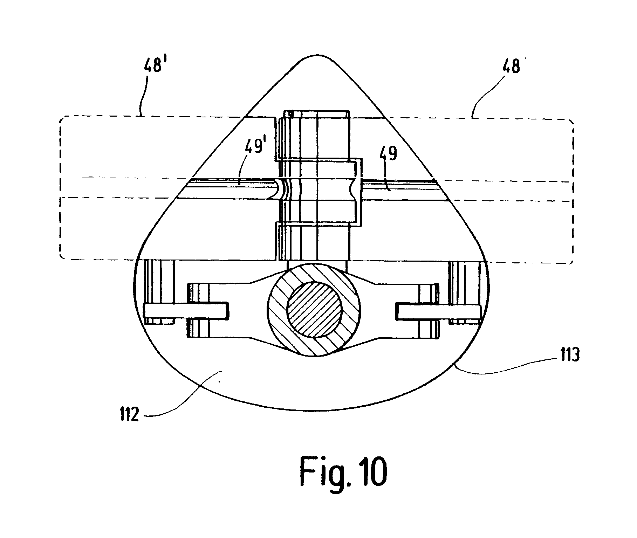

Nunmehr zieht der Operateur das Nahthilfegerät 10 etwas zurück und legt dabei die Flügelplatten 48, 48' an der Unterseite 114 der Bauchdecke 110 an, wie das in Fig. 4 gezeigt ist. Bei dem Übergang von der Schwenkstellung von Fig. 3 zu Fig. 4 überläuft der Zapfen 24 die Flanke bzw. Rampe der Nut 40 und ist wieder in die Nut 36 eingerastet. Dem Operateur zeigt sich die Minilaparotomie 112 von der Außenseite wie in Fig. 10 dargestellt. Von der Außenseite her kann nunmehr ein Haken mit einem Faden, bspw. ein Dechamps-Haken, dessen Ende korkenzieherartig gedreht ist, eingeschoben werden und Nähte bzw. ein Faden 116 gelegt werden, wie dies in Fig. 5 angedeutet ist. Zur Erleichterung der Orientierung des Nahtwerkzeuges dienen Führungsnuten 49, 49' auf den Flügelplatten 48, 48', die der Übersichtlichkeit halber nur in den Fig. 1 und 10 dargestellt sind. Man kann also den Dechamps-Haken, nachdem er jeweils auf die Flügelplatte 48 bzw. die Flügelplatte 48' gestoßen ist, etwas verschieben, bis er in die Nut 49 bzw. 49' eintritt. Das Nahtwerkzeug ist nun ausrichtbar und der Operateur kann beidseits des Schaftes den Faden 116 so legen wie in Fig. 5 angedeutet.

Nach Legen des Fadens 116 wird der Feststellmechanismus 20 erneut entriegelt und der Stab 28 wird über die Fingeröse 30, in die der Daumen eingeschoben ist, nach proximal gezogen.

Die Betätigungshebel 50, 50' drücken nunmehr die Flügelplatten 48, 48' in der Darstellung von Fig. 5 nach unten, diese werden somit distal abgeklappt, und zwar in eine V-förmige Stellung, wie sie in Fig. 5 dargestellt ist. Ein weiteres Abklappen ist nicht notwendig und auch nicht wünschenswert, so daß ausgeschlossen ist, daß zwischen die abgeklappten Flügelplatten 48, 48' Gewebeteile eingeklemmt werden können.

Der Zapfen 24 des Feststellmechanismus gleitet nunmehr längs der Abflachung 32 des Stabes 28.

In der in Fig. 5 dargestellten Stellung kann nunmehr problemlos das Nahthilfegerät 10 von der Minilaparotomie 112 somit vom Körper abgezogen werden.

Anschließend kann der Faden 116 verknotet und somit die Minilaparotomie verschlossen werden.

Zum Erleichtern des Ansetzens und Einbringens des Nahthilfegerätes 10 in die Minilaparotomie 112 ist die in Fig. 11 ersichtliche Führungsschiene 60 vorgesehen.

Die Führungsschiene 60 weist eine Führungsnut 62 auf, an die das Nahthilfegerät 10 wie in Fig. 12 dargestellt angesetzt werden kann. Die Führungsschiene 60 weist einen abgewinkelten Griff 64 auf, so daß beide Geräte, also Führungsschiene 60 und Nahthilfegerät 10 von unterschiedlichen Händen gehalten werden können, ohne sich gegenseitig zu beeinträchtigen. In der Führungsschiene 60 ist eine Skala 68 vorgesehen, über die die Einschubtiefe abgelesen werden kann.

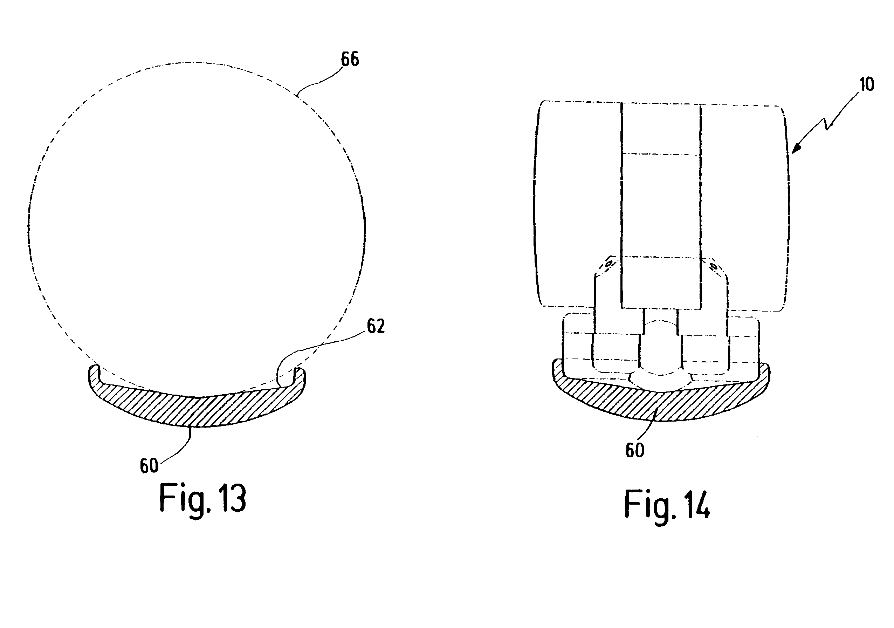

Aus der vergrößerten Schnittdarstellung von Fig. 13 ist zu erkennen, daß die Führungsnut 62 ein bestimmtes Querschnittsprofil aufweist.

Dieses Querschnittsprofil ist so gestaltet, daß die Führungsschiene 16 längs eines Trokars, wie er in Fig. 13 durch die Konturlinie 66 angedeutet ist, in den Körper eingeschoben werden kann.

Nach Einschieben der Führungsschiene 16, wobei die Einschubtiefe über die Skala 68 kontrolliert werden kann, wird der Trokar abgezogen. Anschließend wird das Nahthilfegerät 10 an die Führungsnut 62 angesetzt, wie das in Fig. 12 und 14 dargestellt ist und in den Körper ein- bzw. durch die Minilaparotomie 112 durchgeschoben. Dieses Zusatzgerät erleichtert die Handhabung, insbesondere das atraumatische Ansetzen und Einführen des Nahthilfegerätes 10.

Das zuvor beschriebene Nahthilfegerät 10 ist vollständig aus Metall hergestellt und somit als mehrfach verwendbares Instrument ausgebildet, das autoklavierbar ist.

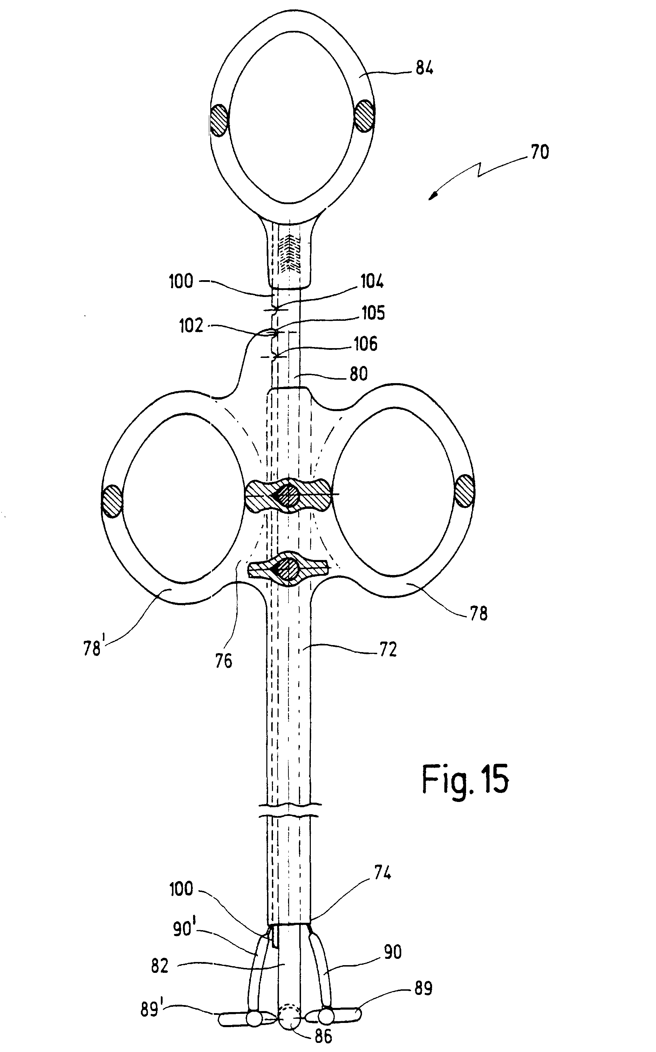

In den Fig. 15 bis 18 ist eine weitere Ausgestaltung eines erfindungsgemäßen Nahthilfegerätes dargestellt, das in seiner Gesamtheit mit der Bezugsziffer 70 versehen ist.

Das Nahthilfegerät 70 ist prinzipiell gleich wie das Nahthilfegerät 10 ausgebildet, jedoch vollständig aus Kunststoff, insbesondere aus Kunststoffspritzteilen, hergestellt, somit kostengünstiger herzustellen und ggf. als Einweggerät einzusetzen.

Das Nahthilfegerät 70 weist dementsprechend einen Schaft 72 auf, der als Rohr 74 ausgebildet ist. Proximal ist das Rohr 74 mit einem Griff 76 versehen, der zwei Fingerösen 78, 78' aufweist.

Im Rohr 74 ist ein Betätigungselement 80 in Form eines längsverschieblichen Stabes 82 aufgenommen, der proximal mit einer Fingeröse 84 versehen ist.

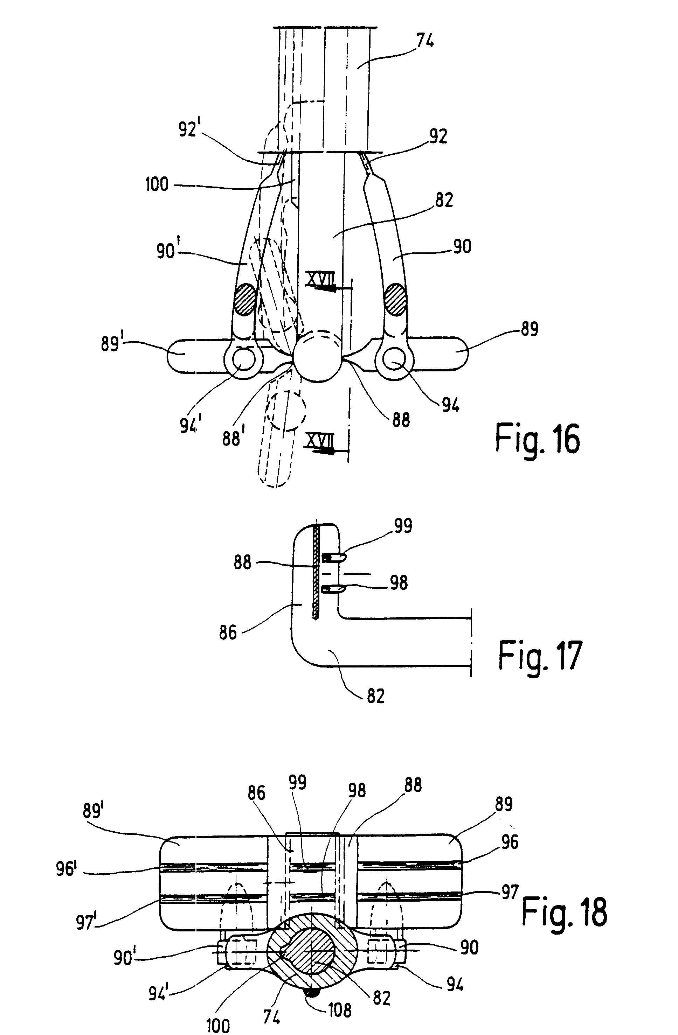

Distal weist der Stab 82 ein abgewinkeltes Ende 86 auf (siehe insbesondere Fig. 17), an dem seitlich über biegsame Kunststoffbrücken 88, 88' die Flügelplatten 89, 89' einstückig angespritzt sind. Die biegsamen Kunststoffbrücken 88, 88' bilden somit Filmscharniere. Zwei Betätigungshebel 90, 90' sind über Kunststoffbrücken 92, 92' (siehe insbesondere Fig. 16) mit dem distalen Ende des Rohrs 74 gelenkig verbunden. Am gegenüberliegenden Ende sind die Betätigungshebel 90, 90' auf gelenkbolzenartige Fortsätze 94, 94' an den Seitenkanten der Flügelplatten 89, 89' aufgeclipst.

Wie insbesondere aus der Darstellung von Fig. 18 ersichtlich ist, sind auf den Flügelplatten 89, 89' zwei längsverlaufende Führungsnuten 96, 97 bzw. 96', 97' vorgesehen.

An entsprechenden Stellen sind auch am abgewinkelten Ende 86 des Stabes 82 entsprechende Führungsnuten 98 und 99 vorgesehen, so daß eine durchgehende Führung vorgesehen ist. Längs der Außenseite des Stabes 82 ist ein Steg 100 in Form eines Angusses angesetzt.

Das Rohr 74 weist eine entsprechende Innenkontur auf, wie das aus der Schnittdarstellung von Fig. 18 ersichtlich ist, somit ist der Stab 82 drehsicher im Rohr 94 aufgenommen.

Am proximalen Ende, vor der Fingeröse 84, sind im Steg 100 Aussparungen 104, 105, 106 vorgesehen, in die eine entsprechende Noppe 102 des Griffes 76 elastisch ein- bzw. ausrasten kann, dazu sind die Aussparungen mit entsprechenden Ein- bzw. Auslaufschrägen versehen. Die drei Aussparungen 104, 105, 106 zeigen bzw. entsprechen den drei Arbeitsstellungen, dementsprechend ist die Noppe 102 in der Darstellung von Fig. 15 in die mittlere Aussparung 105 eingerastet, somit in der Arbeitsposition, in der die Flügelplatten 89, 89' etwa rechtwinklig seitlich ausgeschwenkt sind. Da bei dieser Konstruktion keine Feder vorgesehen sein muß, die den Stab beaufschlagt, werden die Flügelplatten 89, 89' über die Fingeröse 84 in die entsprechenden hochgeklappten bzw. abgeklappten Stellungen bewegt, wobei dann entsprechend die Noppe 102 in die Aussparung 104 bzw. 106 einrastet.

Am distalen Endbereich des Stabes 82 ist ein radial vorspringender Zapfen 108 vorgesehen, der in eine entsprechende Nut in einer Führungsschiene eingesetzt werden kann, um ein definiertes Einschieben des Nahthilfegerätes 70 zu erreichen.

## Patentansprüche

1. Nahthilfegerät für das Verschließen von Minilaparotomien (112) aus minimal-invasiven chirurgischen Eingriffen, gekennzeichnet durch einen Schaft (12, 72), an dessen distalem Ende zumindest eine seitlich ausschwenkbare flächige Auflage (46) angeordnet ist, mit einem Betätigungselement (26, 80) zum Verschwenken der Auflage (46), wobei die Auflage (46) zwischen einer nach proximal hochgeklappten Arbeitsposition, einer seitlich ausgeklappten Arbeitsposition und einer nach distal abgeklappten Arbeitsposition steuerbar ist.

2. Nahthilfegerät nach Anspruch 1, **dadurch gekennzeichnet**, daß die Auflage (46) zwei diametral gegenüberliegende Flügelplatten (48, 48'; 89, 89') aufweist.

3. Nahthilfegerät nach Anspruch 1 oder 2, **dadurch gekennzeichnet**, daß das Betätigungselement (26, 80) als längs des Schaftes (12, 72) verschiebbarer Stab (28, 82) ausgebildet ist, der distal gelenkig mit der Auflage (46) verbunden ist, wobei die Gelenkachse (45) die Schwenkachse der Auflage (46) darstellt.

4. Nahthilfegerät nach einem der Ansprüche 1 bis 3, **dadurch gekennzeichnet**, daß die Auflage (46) im Abstand zu ihrer Schwenkachse (45) gelenkig mit einem Ende eines Betätigungshebels (50, 50'; 90, 90') verbunden ist, dessen anderes Ende gelenkig mit dem Schaft (12, 72) verbunden ist.

5. Nahthilfegerät nach Anspruch 4, **dadurch gekennzeichnet**, daß der Abstand zwischen der Schwenkachse (45) der Auflage (46) und der Anlenkung des Betätigungshebels (50, 50'; 90, 90') etwa einem Drittel der Gesamtlänge der Auflage (46) entspricht.

6. Nahthilfegerät nach einem der Ansprüche 1 bis 5, **dadurch gekennzeichnet**, daß auf der dem proximalen Ende des Nahthilfegerätes (10, 70) zugewandten Seite der Auflage (46) zumindest eine Führung (49, 49'; 96, 96', 97, 97') für ein Nahtwerkzeug vorgesehen ist.

7. Nahthilfegerät nach einem der Ansprüche 1 bis 6, **dadurch gekennzeichnet**, daß das Betätigungselement (26, 80) verdrehsicher im Schaft (12, 72) aufgenommen ist.

8. Nahthilfegerät nach einem der Ansprüche 1 bis 7, **dadurch gekennzeichnet**, daß das Betätigungselement (26) durch die Kraft einer Feder (34) derart beaufschlagt ist, daß die Auflage (46) aus der nach proximal hochgeklappten Arbeitsposition selbständig in Richtung der seitlich ausgeklappten Arbeitsposition bewegt wird.

9. Nahthilfegerät nach Anspruch 8, **dadurch gekennzeichnet**, daß die Kraft der Feder (34) derart eingestellt ist, daß die Arbeitsauflage (46) nur bis in eine Zwischenposition seitlich ausgeklappt wird, wobei die Zwischenposition etwas vor der vollständig seitlich ausgeklappten Arbeitsposition liegt.

10. Nahthilfegerät nach einem der Ansprüche 1 bis 9, **dadurch gekennzeichnet**, daß am Schaft (12, 72) und/oder am Betätigungselement (26, 80) Orientierungsmerkmale (104, 105, 106) vorgesehen sind, die die jeweilige Schwenkstellung der Auflage anzeigen.

11. Nahthilfegerät nach einem der Ansprüche 1 bis 10, **dadurch gekennzeichnet**, daß ein Feststellmechanismus (20) vorgesehen ist, über den die Auflage (46) in bestimmten Schwenkstellungen feststellbar ist.

12. Nahthilfegerät nach Anspruch 11, **dadurch gekennzeichnet**, daß der Feststellmechanismus (20) als ein am Schaft (12, 72) schwenkbar gelagerter Federhebel (22) ausgebildet ist, der ein Rastelement (24, 102) aufweist, das in entsprechende Aussparungen (36, 40; 104, 105, 106) am Betätigungselement (26, 80) eingreift.

13. Nahthilfegerät nach Anspruch 12, **dadurch gekennzeichnet**, daß die Aussparung (36, 40; 104, 105, 106) mit Rampen (38) versehen sind, die ein erkennbares Einrasten verursachen.

14. Nahthilfegerät nach einem der Ansprüche 1 bis 13, **dadurch gekennzeichnet, daß** zu seiner Einführung in eine Minilaparotomie eine spezielle Führungsschiene (60) vorgesehen ist.

15. Nahthilfegerät nach Anspruch 14, **dadurch gekennzeichnet**, daß die Führungsschiene (60) einen abgewinkelten Griff (64) aufweist.

16. Nahthilfegerät nach Anspruch 14 oder 15, **dadurch gekennzeichnet**, daß die Führungsschiene eine Führungsnut für einen Führungszapfen (108) am distalen Ende des Betätigungselementes aufweist, der eine spürbare Führung und Steuerung der Eindringtiefe des Nahthilfegerätes, bezogen auf die Eindringtiefe der Führungsschiene, erlaubt.

17. Nahthilfegerät nach einem der Ansprüche 14 bis 16, **dadurch gekennzeichnet**, daß die Führungsschiene mit einer Skala (68) versehen ist.

## Claims

1. Suturing aid for closing minilaparotomies (112) from minimal-invasive surgical operations, **characterized by** a shaft (12, 72) whose distal end is provided with at least one plane support (46), that can be swung out laterally, and an actuating element (26, 80) for swinging out the support (46), whereby the support (46) can be moved between a working position in which it is folded up toward the proximal end, a working position in which it is folded out laterally, and a working position in which it is folded down toward the distal end.

2. Suturing aid according to claim 1, **characterized in that** said support (46) comprises two diametrically opposite vanes (48, 48'; 89, 89').

3. Suturing aid according to claim 1 or Claim 2, **characterized in that** said actuating element (26, 80) is configured as a rod (28, 82), which is arranged to slide along said shaft (12, 72) and is hinged, on the distal end, on said support (46), with the axis (45) of the hinge forming the pivot axis of said support (46).

4. Suturing aid according to one of claims 1 to 3, **characterized in that** said support (46) is hinged, at a certain distance from its pivot axis (45), on one end of an actuating lever (50, 50'; 90, 90') whose other end is hinged on said shaft (12, 72).

5. Suturing aid according to claim 4, **characterized in that** the distance between said pivot axis (45) of said support (46) and the connection point of said lever (50, 50'; 90, 90') corresponds to approximately one third of the overall length of said support (46).

6. Suturing aid according to anyone of claims 1 to 5, **characterized in that** at least one guide (49, 49'; 96, 96'; 97, 97') for a suturing tool is provided on the side of the support facing the proximal end of said suturing aid (10, 70).

7. Suturing aid according to anyone of claims 1 to 6, **characterized in that** said actuating element (26, 80) is protected against torsion inside said shaft (12, 72).

8. Suturing aid according to anyone of claims 1 to 7, **characterized in that** said actuating element (26) is biased by a spring (34) in such a way that said support (46) is moved automatically from its working position, in which it is folded up toward the proximal end, to its laterally folded-out working position.

9. Suturing aid according to claim 8, **characterized in that** the spring force (34) is adjusted in such a way that said working support (46) will be folded out laterally only to an intermediate position somewhat before the working position in which the support is fully folded out laterally.

10. Suturing aid according to anyone of claims 1 to 9, **characterized in that** orientation marks (104, 105, 106) are provided on said shaft (12, 72) and/or on said actuating element (26, 80) that indicate the angular position which the working support occupies at any time.

11. Suturing aid according to anyone of claims 1 to 10, **characterized in that** a fixing mechanism (20) is provided by means of which said support (46) can be locked in given angular positions.

12. Suturing aid according to claim 11, **characterized in that** said fixing mechanism (20) is configured as a spring lever (22), pivoted on said shaft (12, 72), which comprises a locking element (24, 102) arranged to engage corresponding recesses (36, 40; 104, 105, 106) provided on said actuating element (26, 80).

13. Suturing aid according to claim 12, **characterized in that** said recesses (36, 40; 104, 105, 106) are provided with ramps (38) that provoke a noticeable snap-in effect.

14. Suturing aid according to anyone of claims 1 to 13, **characterized in that** a special guide rail (60) is provided to assist the introduction of the suturing aid into the minilaparotomy.

15. Suturing aid according to Claim 14, **characterized in that** said guide rail (60) comprises a bent-off handle (64).

16. Suturing aid according to claim 14 or claim 15, **characterized in that** said guide rail comprises a guide groove for a guide pin (108) at the distal end of said actuating element, which permits the depth of penetration of the suturing aid, relative to the depth of penetration of the guide rail, to be guided and controlled in a detectable fashion.

17. Suturing aid according to anyone of claims 14 to 16, **characterized in that** the guide rail is provided with a graduation (68).

## Revendications

1. Appareil auxiliaire de couture pour fermer des minilaparotomies (112) dans des interventions chirurgicales le moins invasives possible, **caractérisé** par une tige (12, 72) à l'extrémité distale de laquelle est disposé au moins un support plat (46) pouvant pivoter latéralement vers l'extérieur, avec un élément d'actionnement (26, 80) pour faire pivoter le support (46), le support (46) pouvant être commandé entre une position de travail relevée vers le côté proximal, une position de travail déployée sur le côté et une position de travail rabattue vers le côté distal.

2. Appareil auxiliaire de couture selon la revendication 1, **caractérisé en ce que** le support (46) comporte deux plaques ailettes (48, 48'; 89, 89') diamétralement opposées.

3. Appareil auxiliaire de couture selon la revendication 1 ou 2, **caractérisé en ce que** l'élément d'actionnement (26, 80) est conformé en barre (28, 82) pouvant coulisser le long de la tige (12, 72) et qui est relié côté distal de manière articulée au support (46), l'axe d'articulation (45) constituant l'axe de pivotement du support (46).

4. Appareil auxiliaire de couture selon l'une des revendications 1 à 3, **caractérisé en ce que** le support (46) est relié de manière articulée, à distance de son axe de pivotement (45), à une extrémité d'un levier d'actionnement (50, 50' ; 90, 90') dont l'autre extrémité est reliée de manière articulée à la tige (12, 72).

5. Appareil auxiliaire de couture selon la revendication 4, **caractérisé en ce que** la distance entre l'axe de pivotement (45) et le support (46) et l'articulation du levier d'actionnement (50, 50'; 90, 90') correspond approximativement à un tiers de la longueur totale du support (46).

6. Appareil auxiliaire de couture selon l'une des revendications 1 à 5, **caractérisé en ce que** sur le côté du support (46), tourné vers l'extrémité proximale de l'appareil auxiliaire de couture (10, 70), il est prévu au moins un guide (49, 49' ; 96, 96', 97, 97') pour un outil à coudre.

7. Appareil auxiliaire de couture selon l'une des revendications 1 à 6, **caractérisé en ce que** l'élément d'actionnement (26, 80) est logé dans la tige (12, 72) de manière à pouvoir tourner.

8. Appareil auxiliaire de couture selon l'une des revendications 1 à 7, **caractérisé en ce que** l'élément d'actionnement (26) est soumis à la force d'un ressort (34) de manière que le support (46) soit automatiquement déplacé, depuis la position de travail rabattue vers le haut, vers le côté proximal, en direction de la position de travail rabattue ouverte sur le côté.

9. Appareil auxiliaire de couture selon la revendication 8, **caractérisé en ce que** la force du ressort (34) est réglée de manière que le support de travail (46) ne soit rabattu ouvert sur le côté que jusque dans une position intermédiaire, la position intermédiaire se situant un peu devant la position de travail totalement rabattue ouverte sur le côté.

10. Appareil auxiliaire de couture selon l'une des revendications 1 à 9, **caractérisé en ce que** sur la tige (12, 72) et/ou l'élément d'actionnement (26, 80) sont prévues des caractéristiques d'orientation (104, 105, 106) qui indiquent chaque position de pivotement du support.

11. Appareil auxiliaire de couture selon l'une des revendications 1 à 10, **caractérisé en ce qu**'il est prévu un mécanisme de blocage (20) par lequel le support (46) peut être bloqué dans des positions de pivotement déterminées.

12. Appareil auxiliaire de couture selon la revendication 11, **caractérisé en ce que** le mécanisme de blocage (20) est conformé en levier élastique (22), monté pivotant sur la tige (12, 72) et qui comporte un élément d'encliquetage (24, 102), lequel s'engage dans des découpes (36, 40; 104, 105, 106) correspondantes de l'élément d'actionnement (26, 80).

13. Appareil auxiliaire de couture selon la revendication 12, **caractérisé en ce que** la découpe (36, 40; 104, 105, 106) est pourvue de rampes (38) qui provoquent un encliquetage reconnaissable.

14. Appareil auxiliaire de couture selon l'une des revendications 1 à 13, **caractérisé en ce qu**'il est prévu pour son introduction dans une minilaparotomie, un rail de guidage (60) particulier.

15. Appareil auxiliaire de couture selon la revendication 14, **caractérisé en ce que** le rail de guidage (60) comporte une poignée coudée (64).

16. Appareil auxiliaire de couture selon la revendication 14 ou 15, **caractérisé en ce que** le rail de guidage présente une rainure de guidage pour un tenon de guidage (108) à l'extrémité distale de l'élément d'actionnement, lequel tenon permet un guidage sensible et une commande de la profondeur de pénétration de l'appareil auxiliaire de couture, par rapport à la profondeur de pénétration du rail de guidage.

17. Appareil auxiliaire de couture selon l'une des revendications 14 à 16, **caractérisé en ce que** le rail de guidage est pourvu d'une échelle (68).
